# EUROPEAN PATENT SPECIFICATION

(11) **EP 0 449 232 B1**
(45) Date of publication and mention of the grant of the patent: **21.12.1994**
(21) Application number: 91104810.6
(22) Date of filing: 26.03.1991
(51) Int. Cl.: A61G 11/00

(54) **Infant incubator**
Säuglingsinkubator
Couveuse pour nouveau-nés

(30) Priority: 26.03.1990 US 499091
(43) Date of publication of application: 02.10.1991
(73) Proprietor: AIR-SHIELDS, INC., Hatboro, PA 19040 (US)
(72) Inventor: Moffett, Joseph J., Furlong, Pennsylvania 18925 (US); Grosholz, James R., New Hope, Pennsylvania 18938 (US)
(74) Representative: Grünecker, Kinkeldey, Stockmair & Schwanhäusser Anwaltssozietät

(56) References cited:
- DE-A- 2 944 508
- US-A- 3 335 713
- US-A- 4 346 701
- US-A- 4 796 605

## Description

### TECHNICAL FIELD

The present invention relates, in general, to infant incubators and, in particular, to an infant incubator having a humidifier arranged to greatly reduce or eliminate entirely the tendency, if any, of air borne contaminants affecting the quality of the air flowing through the area in which an infant is resting.

### BACKGROUND OF THE INVENTION

Generally, inlet air to an infant incubator is filtered before the air enters the circulation path which extends through the hood area in which an infant is maintained and treated. Typically, the filtered air is drawn by a fan in the circulation path and conducted into the hood through a first passage. The air leaves the hood through a second passage and is conducted back to the first passage for recirculation through the hood. A heater and a humidifier are located in the air circulation path, so that the air which is introduced into the hood has the proper temperature and humidity.

The hood of an infant incubator usually is arranged with arm ports and a door to permit access to the infant if the need arises to treat the infant or to position sensors, probes and the like on the infant. When personnel attending an infant open the hood door or place their arms through the arm ports, contaminants can be introduced from the hands of such personnel and, to a lesser extent, from the environment outside the incubator.

Many users of infant incubators have the perception that such contaminants, which become airborne and enter the air circulation path, are the origin of airborne bacteria formed in the reservoir of the humidifier as the air flow with the contaminants passes through the humidifier, whereby the infant is exposed to this bacteria. Although this perception has not been substantiated, many users who have this perception, nevertheless, do not make use of the humidifier, for example, leaving the reservoir empty. Instead, they employ external humidifiers which introduce filtered or unfiltered, humidified air directly into the hood of the incubator. If the incoming humidified air from an external humidifier is not filtered appropriately, this air can carry its own variety of airborne pathogens if the humidifier reservoir is not rigorously maintained.

Such external humidifiers have a number of shortcomings. First, they require a source of pressurized gas (oxygen, air or oxygen/air mixture) to force humidified air into the incubator hood. Second, provision must be made for passing through the hood tubing and conduits through which the humidified air is conducted into the hood. Third, the presence of such tubing and conduits can impede the maintenance and treatment of an infant within the hood. Fourth, tubing and conduits leading into the incubator hood have a propensity for a temperature drop across their lengths which can result in water vapor condensing back into a liquid state. Water in the tubing and conduits can be absorbed by linens in the incubator, thus increasing the possibility of bacteria colonization in both the tubing and conduits and the absorbent materials in the incubator. Fifth, external humidifiers generally are mounted on IV poles and the like which take up space in already crowded nurseries.

In an infant incubator, as known from DE-A-29 44 508, inlet air to the incubator is filtered before the air enters the circulation path crossing the space below the hood under which an infant is maintained and treated. Filtered air is drawn into the circulation path by a fan and is conducted into the space through a first opening. The air leaves the space through a second opening and is conducted back to the first opening for recirculation. A heater and a humidifier are located in the main-air-recirculation path so that the air which is introduced into the space has the proper temperature and humidity. Contaminants which have become air borne and enter the air circulation path are the origin of air borne bacteria formed in the reservoir of the humidifier as the air flow with the contaminants passes through the humidifier. An infant within the incubator becomes exposed to the bacteria. Any comtaminants introduced into the space of the incubator are collected in the humidifier.

### SUMMARY OF THE INVENTION

Accordingly, an infant incubator constructed in accordance with the present invention includes a hood and a base upon which the hood is mounted. Also included are first air passage means extending between first and second openings into the space defined by the hood for conducting air flow into this space through the first opening and for conducting air flow from the space within the hood through the second opening and to the first opening. This infant incubator further includes second air passage means opening to the atmosphere outside the incubator for receiving inlet air and means within the second air passage means for filtering inlet air. Also included are means for humidifying filtered inlet air and for conducting humidified filtered inlet air to the first air passage means and means for drawing humidified filtered inlet air into the first air passage means and for circulating air flow through the hood and the first air passage means.

### BRIEF DESCRIPTION OF THE DRAWINGS

Figure 1 is a schematic block diagram of an infant incubator constructed in accordance with the present invention;
Figure 2 is a sectional view of a portion of the air inlet line of a commercially available infant incubator; and
Figures 3 and 4 are sectional views which illustrate the manner in which the air inlet line of the infant incubator of Figure 2 can be modified to selectively place a humidifier in the air flow path of filtered inlet air in accordance with the present invention.

### DETAILED DESCRIPTION OF THE INVENTION

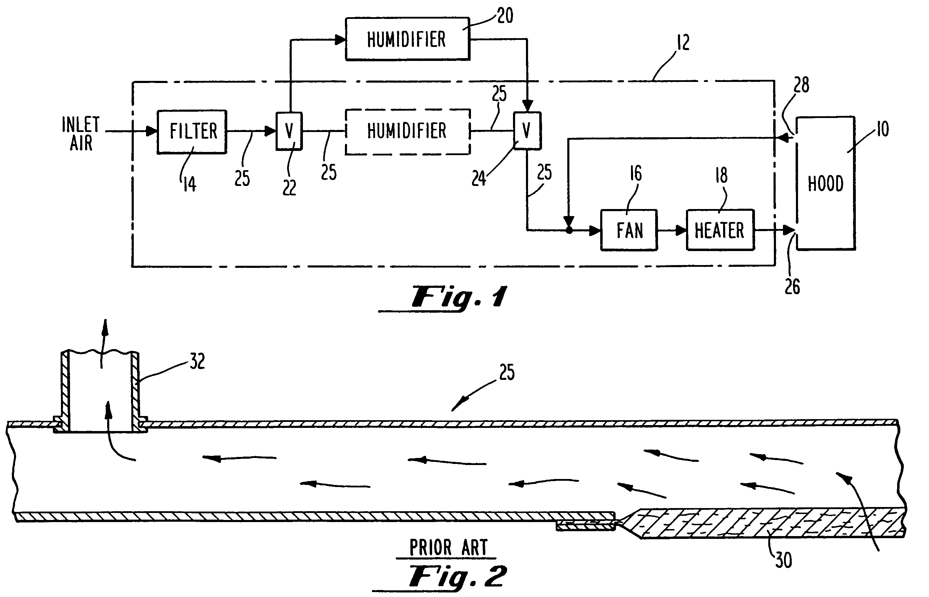

An infant incubator, constructed in accordance with the present invention and illustrated schematically in Figure 1, includes a hood 10 and a base upon which the hood is mounted. The base is represented in Figure 1 by the dot-dash box identified by reference numeral 12 and contains an inlet air filter 14, a fan 16, a heater 18 and air passages for conducting filtered inlet air to the hood and recirculating air from the hood. U.S. 3,335,713 is incorporated herein by reference to supplement the disclosure of an incubator hood mounted on a base which contains various components, except for a humidifier.

An infant incubator, constructed in accordance with the present invention, also includes means for humidifying the filtered inlet air before it reaches the air circulation path. Such means include a humidifier 20 and a pair of valves 22 and 24 which divert filtered inlet air from the air passage between the valves to the humidifier when humidification of the filtered inlet air is desired. Humidifier 20 and its associated air passages, in effect, form a bypass to the air inlet line 25. This arrangement of the humidifier is particularly suited for adapting certain types of infant incubators already in service to incorporate the present invention. In an alternate arrangement of the present invention, shown by the dashed lines in Figure 1, the humidifier can be located in the air inlet line 25 and without valves 22 and 24.

In operation, inlet air is filtered by filter 14 and the filtered inlet air is diverted by valve 22 to humidifier 20 where it is humidified to the desired extent. The humidified air is conducted through valve 24 to air passage means which extend between first and second openings 26 and 28 into the space defined by hood 10. The air passage means conduct air flow into hood 10 through opening 26 and conduct air flow from the hood through opening 28 back to opening 26. Fan 16, located in the air passage means, draws the humidified filtered inlet air to the air passage means and circulates air through the hood and the air passage means. Hood 10 is not an air-tight container. Rather, air, equal in volume to the air taken in at the air inlet, exits from the hood either by leakage at joints or seams in the incubator construction or through openings provided for the purpose of letting air escape. Heater 18, also located in the air passage means, heats the air introduced into the hood to the proper temperature.

It is important to note that the air is not humidified by humidifying apparatus located in the air circulation path. Consequently, air borne contaminants, if any, introduced in hood 10 have no water reservoir in which to collect and form bacteria which might become air borne and enter the hood when the air is circulated back into the hood. Rather, filtered inlet air is humidified upstream of the air circulation path, so that any contaminants introduced in hood 10 do not collect in the humidifier. For incubators in service having humidifying units in the air circulation path, this result can be achieved simply by leaving the humidifier reservoir empty which, as indicated above, is the way many users operate such equipment when external humidifiers are used. This is represented in Figure 1 by the absence of a humidifier in the air circulation path.

Thus, the present invention, as depicted schematically in Figure 1, provides an effective and practical answer to the unsubstantiated perception of many users of incubators that contaminants introduced in the hood of the incubator result in air borne bacteria circulated into the hood. The solid line arrangement of humidifier 20, whereby the humidifier forms a bypass to the air inlet line, is particularly useful in retrofitting incubators in service to achieve the desired result.

Referring to Figure 2, which is a sectional view of a portion of the air inlet line 25 of a commercially available infant incubator, inlet air, shown by the arrows, enters the incubator through a filter pad 30. The filtered inlet air flows along air inlet line 25 and through a coupling 32 to the circulation path which is not shown in Figure 2 but is shown in Figure 1. There is no valve, such as valve 24, through which the humidified filtered inlet air passes from air inlet line 25 to the air circulation path.

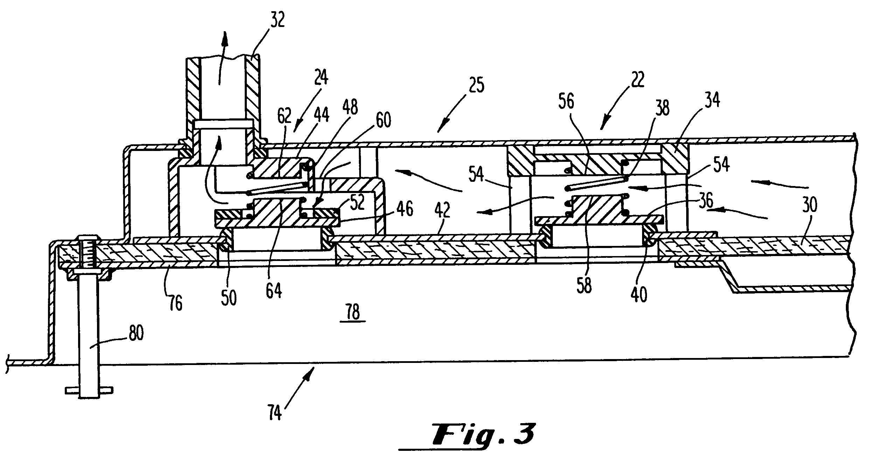

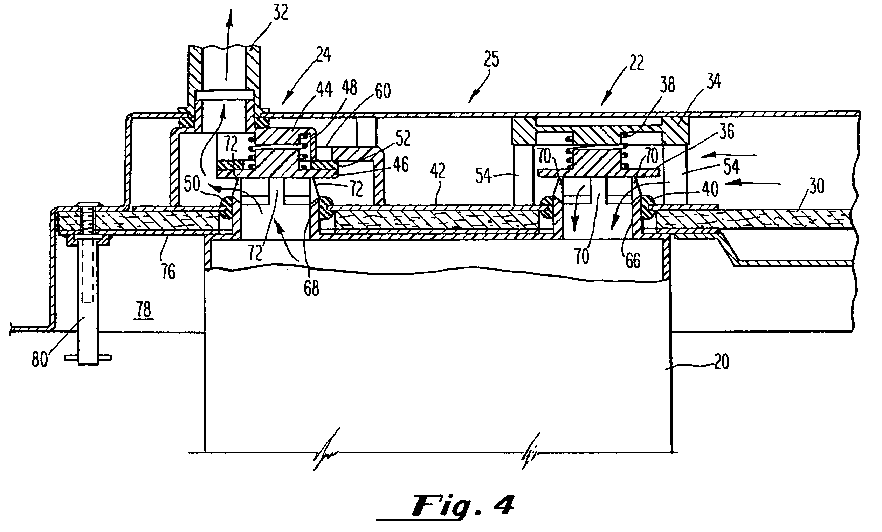

Figures 3 and 4, which are sectional views, illustrate the manner in which air inlet line 25 of Figure 2 can be modified to selectively place a humidifier in the air flow path of the filtered inlet air as shown schematically in Figure 1. Figure 3 illustrates the air inlet line before the humidifier is introduced, while Figure 4 illustrates the humidifier in place.

Referring to Figures 3 and 4, valve 22 of Figure 1 is composed of a base 34 fixed in position within air inlet line 25, a cap 36 movable transverse to air inlet line 25, and a spring 38 which urges cap 36 against a sealing grommet 40 fitted in a first opening in a wall 42 of air inlet line 25. Valve 24 of Figure 1 is composed of a base 44 fixed in position within air inlet line 25, a cap 46 movable transverse to air inlet line 25, a spring 48 which urges cap 46 against a sealing grommet 50 fitted in a second opening in wall 42, and a sealing ring 52 which is carried on movable cap 46.

With valves 22 and 24 as shown in Figure 3, namely without the humidifier installed, filtered inlet air flows through valve 22, air inlet line 25 and valve 24 to coupling 32 and the air circulation path. In particular, base 34 of valve 22 has three legs 54 (only two are shown in Figures 3 and 4). Filtered inlet air flows into and through valve 22 and then into and through valve 24 by passing through an opening 60 in base 44 of valve 24. After passing through valve 24, the filtered inlet air passes through coupling 32 to the air circulation path. With caps 36 and 46 of valves 22 and 24, respectively, pressed against sealing grommets 40 and 50, respectively, the two openings in wall 42 of air inlet line 25 are sealed.

When humidifier 20 is installed, as shown in Figure 4, an inlet port coupling 66 to the humidifier and an outlet port coupling 68 from the humidifier engage caps 36 and 46, respectively, of valves 22 and 24, respectively, to move caps 36 and 46 against the action of springs 38 and 48, respectively, and away from sealing grommets 40 and 50, respectively. Inlet port coupling 66 and outlet port coupling 68 each are arranged with three prongs 70 and 72, respectively. This permits filtered inlet air to enter humidifier 20 through inlet port coupling 66 and humidified air to leave the humidifier through outlet port coupling 68. The humidified air passes through coupling 32 to the air circulation path. With the humidifier installed, unhumidified filtered inlet air continues to pass through valve 22. However, as shown in Figure 4, sealing ring 52, carried on cap 46, seals opening 60 in base 44 to close valve 24, thereby preventing unhumidified filtered inlet air from flowing to coupling 32.

Figures 3 and 4 illustrate other modifications to the air inlet line of Figure 2 besides those already described. Filter pad 30 is longer and extends beyond the outlet from the humidifier, with openings in the filter pad at the inlet to the humidifier and the outlet from the humidifier. A member 74 having a vertical wall 76 and a base 78 serves to clamp filter pad 30 in place between wall 42 and wall 76 and as a support surface for the humidifier on base 78. Member 74 is mounted on air inlet line 25 by suitable means, such as a stud arrangement 80.

A humidifier which is particularly suited for incorporation in an infant incubator constructed in accordance with the present invention is known from EP-A-0 449 233. However, the present invention can incorporate other types of humidifiers and, as indicated by Figure 1 and the associated text, the present invention is applicable to infant incubators in which the humidifier is permanently installed.

## Claims

1. An infant incubator comprising:
a hood (10), a base (12) upon which said hood is mounted, first air passage means (16, 18) extending between first and second openings (26, 28) into said hood for conducting air flow into said hood through said first opening and for conducting air flow leaving said hood through said second opening and to said first opening, means for supplying inlet air , second air passage means (25) for conducting inlet air to said first air passage means, means (14) within said second air passage means for filtering inlet air, and means (16) within said first air passage means for drawing humidified filtered inlet air or unhumidified filtered inlet air into said first air passage means and for circulating air flow through said hood and said first air passage means,
characterized by humidifying means (20, 22, 24) upstream from said first air passage means for selectively humidifying filtered inlet air and for selectively conducting humidified filtered inlet air or unhumidified filtered inlet air to said first air passage means.

2. An infant incubator according to claim 1 characterized by said humidifying means including a humidifier (20), first valve means (22) in said second air passage means for conducting filtered inlet air to said humidifier, and second valve means (24) in said second air passage means for conducting humidified filtered inlet air from said humidifier to said first air passage means.

3. An infant incubator according to claim 1 characterized by said humidifying means (20, 22, 24) also selectively preventing unhumidified filtered inlet air from being conducted to said first air passage means (16, 18) upon selection.

4. An infant incubator according to claim 1 characterized by said second air passage means opening (25) to the atmosphere for receiving inlet air from the atmosphere.

5. An infant incubator according to claim 2 characterized by said humidifier (20) including means (66, 68) for controlling said first valve means (22) and said second valve means (24).

6. An infant incubator according to claim 5 characterized by said humidifier (20) having an inlet port coupling (66) to said humidifier and an outlet port coupling (68) from said humidifier,
by said first valve means (22) conducting filtered inlet air from said second air passage means (25) to said humidifier in response to engagement of said first valve means by said inlet port coupling to said humidifier, and
by said second valve means (24) conducting humidified filtered inlet air from said second air passage means to said first air passage means (16, 18) in response to engagement of said second valve means by said outlet port coupling from said humidifier.

7. An infant incubator according to claim 6 characterized by said second air passage means (25) including an air inlet line (25) having spaced apart first and second openings in a wall thereof and first and second sealing grommets (40, 50) fitted in said first and second openings, respectively, of said air inlet line,
by said first valve means (22) including a base (34) fixed in position in said air inlet line, a cap (36) movable transverse to said air inlet line, and a spring (38) urging said cap of said first valve means against said first grommet (40) to seal said first opening in said air inlet line, and
by said second valve means (24) including a base (44) fixed in position in said air inlet line, a cap (46) movable transverse to said air inlet line, a spring (48) urging said cap of said second valve means against said second grommet (50) to seal said second opening in said air inlet line, and a sealing ring (52) carried by said cap of said second valve means.

8. An infant incubator according to claim 7 characterized by said humidifier (20) including means (66) for engaging said cap (36) of said first valve means (22) to control said first valve means and means (68) for engaging said cap (46) of said second valve means (24) to control said second valve means.

9. An infant incubator according to claim 8 characterized by said inlet port coupling (66) to said humidifier (20) adapted to engage said cap (36) of said first valve means (22) to move said cap of said first valve means away from said first grommet (40) against the action of said spring (38) of said first valve means and said outlet port coupling (68) from said humidifier adapted to engage said cap (46) of said second valve means (24) to move said cap or said second valve means away from said second grommet (50) against the action of said spring (48) of said second valve means and to move said sealing ring (52) of said second valve means to seal said opening in said base (44) of said second valve means.

10. An infant incubator according to claim 1 characterized by said humidifying means including a humidifier (20) which is selectively mounted to said base (12), and
by first and second valve means (22, 24) upstream from said first air passage means (16, 18) for selectively diverting filtered inlet air from said second air passage means (25) to said humidifier or for passing filtered inlet air through said second air passage means to said first air passage means and for introducing humidified filtered inlet air to said first air passage means.

11. An infant incubator according to claim 10 characterized by said humidifier (20) having valve control means (66, 68) for controlling said first and said second valve means (22, 24) to block flow of filtered inlet air through said second air passage means (25) and conduct filtered inlet air to said humidifier when said humidifier is mounted to said base (12) and introduce humidified filtered inlet air to said first air passage means (16, 18) when said humidifier is mounted to said base.

## Patentansprüche

1. Baby-Inkubator mit
einer Haube (10), einem Grundkörper (12), auf dem die Haube montiert ist, ersten Luftdurchgangseinrichtungen (16,18), die sich zwischen ersten und zweiten Öffnungen (26,28) zur Haube zum Einleiten einer Luftströmung in die Haube durch die erste Öffnung und zum Führen des die Haube verlassenden Luftstroms durch die zweite Öffnung und zu der ersten Öffnung erstrecken,
Einrichtungen zum Zuführen von Einlaßluft, zweiten Luftdurchgangseinrichtungen (25) zum Führen von Einlaßluft zu den ersten Luftdurchgangseinrichtungen,
Einrichtungen (14) innerhalb der zweiten Luftdurchgangseinrichtungen zum Filtrieren der Einlaßluft, und
Einrichtungen (16) innerhalb der ersten Luftdurchgangseinrichtungen zum Ansaugen befeuchteter gefilterter Einlaßluft oder unbefeuchteter gefilterter Einlaßluft in die ersten Luftdurchgangseinrichtungen und zum Zirkulieren eines Luftstroms durch die Haube und die ersten Luftdurchgangseinrichtungen,
**gekennzeichnet durch**
Befeuchtungseinrichtungen (20,22,24) stromab der ersten Luftdurchgangseinrichtungen (16,18) zum wahlweisen Befeuchten gefilterter Einlaßluft und zum wahlweisen Überführen befeuchteter gefilterter Einlaßluft oder unbefeuchteter gefilterter Einlaßluft zu den ersten Luftdurchgangsmitteln (16,18).

2. Baby-Inkubator gemäß Anspruch 1, **dadurch gekennzeichnet,** daß die Befeuchtungseinrichtungen (20,22,24) einen Befeuchter (20), erste Ventileinrichtungen (22) in den zweiten Luftdurchgangseinrichtungen (25) zum Zuleiten gefilterter Einlaßluft zum Befeuchter und zweite Ventileinrichtungen (24) in den zweiten Luftdurchgangseinrichtungen (25) aufweisen, um befeuchtete gefilterte Einlaßluft von dem Befeuchter zu den ersten Luftdurchgangseinrichtungen zu leiten.

3. Baby-Inkubator gemäß Anspruch 1, **dadurch gekennzeichnet,** daß die Befeuchtungseinrichtungen (20,22,24) ferner wahlweise verhindern, daß nicht befeuchtete gefilterte Einlaßluft zu den ersten Luftdurchgangseinrichtungen (16,18) geleitet wird.

4. Baby-Inkubator gemäß Anspruch 1, **dadurch gekennzeichnet,** daß die zweiten Luftdurchgangseinrichtungen (25) sich zur Atmosphäre öffnen, um Einlaßluft aus der Atmosphäre aufzunehmen.

5. Baby-Inkubator gemäß Anspruch 2, **dadurch gekennzeichnet,** daß die Befeuchtungseinrichtungen (20) Einrichtungen (66,68) zum Steuern der ersten Ventilmittel (22) und der zweiten Ventilmittel (24) aufweisen.

6. Baby-Inkubator gemäß Anspruch 5, **dadurch gekennzeichnet,** daß die Befeuchtungseinrichtungen (20) eine Einlaßöffnungskupplung (66) zum Befeuchter und eine Auslaßöffnungskupplung (68) aus dem Befeuchter aufweisen,
daß die ersten Ventileinrichtungen (22) gefilterte Einlaßluft von den zweiten Luftdurchgangseinrichtungen (25) zum Befeuchter unter Ansprechen auf einen Eingriff zwischen der Einlaßöffnungskupplung des Befeuchters und den ersten Ventileinrichtungen leiten, und
daß die zweiten Ventileinrichtungen (24) befeuchtete gefilterte Einlaßluft von den zweiten Luftdurchgangseinrichtungen zu den ersten Luftdurchgangseinrichtungen (16,18) unter Ansprechen auf einen Eingriff zwischen den zweiten Ventileinrichtungen und der Auslaßöffnungskupplung des Befeuchters leiten.

7. Baby-Inkubator gemäß Anspruch 6, **dadurch gekennzeichnet,** daß die zweiten Luftdurchgangseinrichtungen (25) eine Lufteinlaßleitung (25) umfassen, die voneinander beabstandete erste und zweite Öffnungen in einer Wand der Einlaßleitung und erste und zweite Dichtungstüllen (40,50) aufweist, die in den ersten und zweiten Öffnungen der Lufteinlaßleitung angebracht sind,
daß die ersten Ventileinrichtungen (22) einen in seiner Position in der Lufteinlaßleitung festgelegten Grundteil (34), eine quer zur Lufteinlaßleitung bewegbare Kappe (36) und eine Feder (38) aufweisen, die die Kappe der ersten Ventileinrichtungen gegen die erste Dichtungstülle (40) beaufschlagen, um die erste Öffnung der Lufteinlaßleitung abzusperren, und
daß die zweiten Ventileinrichtungen (24) einen in der Lufteinlaßleitung in seiner Position festgelegten Grundteil (44), eine quer zur Lufteinlaßleitung bewegliche Kappe (46) und eine Feder (48) aufweisen, die die Kappe der zweiten Ventileinrichtungen gegen die zweite Dichtungstülle (50) beaufschlagen, um die zweite Öffnung in der Lufteinlaßleitung abzusperren, und daß an der Kappe (46) der zweiten Ventileinrichtungen ein Dichtungsring (52) angebracht ist.

8. Baby-Inkubator gemäß Anspruch 7, **dadurch gekennzeichnet,** daß die Befeuchtungseinrichtungen (20) Einrichtungen (66) zum Angreifen an der Kappe (36) der ersten Ventileinrichtungen (22) aufweisen, um die ersten Ventileinrichtungen zu steuern, und daß die Befeuchtungseinrichtungen (20) Mittel (66) aufweisen, mit denen zum steuern der zweiten Ventileinrichtungen an der Kappe (46) der zweiten Ventileinrichtungen (24) angreifbar ist.

9. Baby-Inkubator gemäß Anspruch 8, **dadurch gekennzeichnet,** daß die Einlaßöffnungskuplung (66) des Befeuchters (20) so ausgebildet ist, daß sie an der Kappe (36) der ersten Ventileinrichtungen (22) zum Bewegen der Kappe von der ersten Dichtungstülle (40) weg und gegen die Beaufschlagung der Feder (38) der ersten Ventileinrichtungen ausgebildet ist, und daß die Auslaßöffnungskupplung (68) des Befeuchters so ausgebildet ist, daß sie zum Angriff an der Kappe (46) der zweiten Ventileinrichtungen (24) zum Bewegen der Kappe der zweiten Ventileinrichtungen von der zweiten Dichtungstülle (50) weg und gegen die Beaufschlagung der Feder (48) der zweiten Ventileinrichtungen und zum Bewegen des Dichtungsringes (52) der zweiten Ventileinrichtungen zum Abschließen der Öffnung im Grundteil (44) der zweiten Ventileinrichtungen bringbar ist.

10. Baby-Inkubator gemäß Anspruch 1, **dadurch gekennzeichent,** daß die Befeuchtungseinrichtungen einen Befeuchter umfassen, der wahlweise an den Grundkörper (12) montierbar ist, und daß stromauf der ersten Luftdurchgangseinrichtungen (16,18) erste und zweite Ventileinrichtungen (22,24) vorgesehen sind, um wahlweise gefilterte Einlaßluft entweder von den zweiten Luftdurchgangseinrichtungen (25) zum Befeuchter oder gefilterte Einlaßluft durch die zweiten Luftdurchgangseinrichtungen zu den ersten Luftdurchgangseinrichtungen und befeuchtete gefilterte Einlaßluft in die ersten Luftdurchgangseinrichtungen zu leiten.

11. Baby-Inkubator gemäß Anspruch 10, **dadurch gekennzeichnet,** daß die Befeuchtungseinrichtungen (20) Steuereinrichtungen (66,68) zum Steuern der ersten und zweiten Ventileinrichtungen (22,24) aufweisen, um eine Strömung gefilterter Einlaßluft durch die zweiten Luftdurchgangseinrichtungen (25) zu blockieren und gefilterte Einlaßluft dem Befeuchter zuzuführen, sobald der Befeuchter am Grundkörper (12) montiert ist, und um ferner befeuchtete gefilterte Einlaßluft den ersten Luftdurchgangseinrichtungen (16,18) zuzuleiten, sobald der Befeuchter an dem Grundkörper (12) montiert ist.

## Revendications

1. Une couveuse pour nouveau-nés comprenant :
une cloche (10), un socle (12) sur lequel est montée la cloche, un premier moyen de passage d'air (16, 18) situé entre les première et seconde ouvertures (26, 28) en communication avec la cloche pour conduire le flux d'air dans la cloche par la première ouverture et pour faire sortir le flux d'air de la cloche par la seconde ouverture et le ramener à la première ouverture, un moyen pour fournir l'air d'admission , un second moyen de passage d'air (25) pour conduire l'air d'admission au premier moyen de passage d'air, un moyen (14) situé dans le second moyen de passage d'air pour filtrer l'air d'admission, et un moyen (16) situé dans le premier moyen de passage d'air pour aspirer l'air d'admission filtré humidifié ou l'air d'admission filtré non humidifié dans le premier moyen de passage d'air et pour faire circuler le flux d'air dans la cloche et le premier moyen de passage d'air.
caractérisée par un moyen d'humidification (20, 22, 24) en amont du premier moyen de passage d'air pour humidifier sélectivement l'air d'admission filtré et pour conduire sélectivement l'air d'admission filtré humidifié ou l'air d'admission filtré non humidifié au premier moyen de passage d'air.

2. Une couveuse pour nouveau-nés selon la revendication 1 caractérisée en ce que le moyen d'humidification comprend un humidificateur (20), une première vanne (22) située dans le second moyen de passage d'air pour conduire l'air d'admission filtré à l'humidificateur, et une seconde vanne (24) située dans le second moyen de passage d'air pour conduire l'ait d'admission filtré humidifié de l'humidificateur au premier moyen de passage d'air.

3. Une couveuse pour nouveau-nés selon la revendication 1 caractérisée en ce que le moyen d'humidification (20, 22, 24) a également pour fonction d'empêcher sélectivement l'admission d'air filtré non humidifié d'être conduit au premier moyen de passage d'air (16, 18) après sélection.

4. Une couveuse pour nouveau-nés selon la revendication 1 caractérisée en ce que le second moyen de passage d'air (25) est en communication avec l'atmosphère pour recevoir l'air d'admission de l'atmosphère.

5. Une couveuse pour nouveau-nés selon la revendication 2 caractérisée en ce que l'humidificateur (20) comprend des moyens (66, 68) pour commander la première vanne (22) et la seconde vanne (24).

6. Une couveuse pour nouveau-nés selon la revendication 5 caractérisée en ce que l'humidificateur (20) a un raccord d'orifice d'entrée (66) dans l'humidificateur et un raccord d'orifice de sortie (68) de l'humidificateur
en ce que la première vanne (22) conduit l'air d'admission filtré du second moyen de passage d'air (25) à l'humidificateur lorsque la première vanne est reliée à l'humidificateur par le raccord d'orifice d'entrée, et
en ce que la seconde vanne (24) conduit l'air d'admission filtré humidifié du second moyen de passage d'air au premier moyen de passage d'air (16, 18) lorsque la seconde vanne est reliée à l'humidificateur par le raccord d'orifice de sortie.

7. Une couveuse pour nouveau-nés selon la revendication 6 caractérisée en ce que le second moyen de passage d'air (25) comprend une conduite d'admission d'air (25) munie dans une de ses parois d'une première et d'une seconde ouvertures distantes l'une de l'autre et d'une première et d'une seconde rondelles d'étanchéité (40, 50) respectivement montées dans les première et seconde ouvertures de la conduite d'admission d'air,
en ce que la première vanne (22) comprend un socle (34) fixé dans la conduite d'admission d'air, un chapeau (36) mobile transversalement par rapport à la conduite d'admission d'air, et un ressort (38) poussant le chapeau de la première vanne contre la première rondelle (40) pour fermer hermétiquement la première ouverture de la conduite d'admission d'air, et
en ce que la seconde vanne (24) comprend un socle (44) fixé dans la conduite d'admission d'air, un chapeau (46) mobile transversalement par rapport à la conduite d'admission d'air, un ressort (48) poussant le chapeau de la seconde vanne contre la seconde rondelle (50) pour fermer hermétiquement la seconde ouverture de la conduite d'admission d'air, et un anneau d'étanchéité (52) porté par le chapeau de la seconde vanne.

8. Une couveuse pour nouveau-nés selon la revendication 7 caractérisée en ce que l'humidificateur (20) comprend un moyen (66) s'engageant dans la chapeau (36) de la première vanne (22) pour commander la première vanne et un moyen (68) s'engageant dans le chapeau (46) de la seconde vanne (24) pour commander la seconde vanne.

9. Une couveuse pour nouveau-nés selon la revendication 8 caractérisée en ce que le raccord d'orifice d'entrée (66) dans l'humidificateur (20) est adapté de façon à s'engager dans le chapeau (36) de la première vanne (22) pour écarter de la première rondelle (40) le chapeau de la première vanne contre l'action du ressort (38) de la première vanne et en ce que le raccord d'orifice de sortie (68) de l'humidificateur est adapté de façon à s'engager dans le chapeau (46) de la seconde vanne (24) pour écarter de la seconde rondelle (50) le chapeau de la seconde vanne contre l'action du ressort (48) de la seconde vanne et pour déplacer l'anneau d'étanchéité (52) de la seconde vanne afin de fermer hermétiquement l'ouverture pratiquée dans le socle (44) de la seconde vanne.

10. Une couveuse pour nouveau-nés selon la revendication 1 caractérisée en ce que le moyen d'humidification comprend un humidificateur (20) qui est monté sélectivement sur le socle (12), et
en ce qu'une première et une seconde vannes (22, 24) sont situées en amont du premier moyen de passage d'air (16, 18) pour dévier sélectivement l'air d'admission filtré du second moyen de passage d'air (25) vers l'humidificateur ou pour conduire l'air d'admission filtré au premier moyen de passage d'air via le second moyen de passage d'air et introduire l'air d'admission filtré humidifié dans le premier moyen de passage d'air.

11. Une couveuse pour nouveau-nés selon la revendication 10 caractérisée en ce que l'humidificateur (20) a des moyens de commande de vannes (66, 68) pour commaander les première et seconde vannes (22, 24) pour stopper l'écoulement de l'air d'admission filtré dans le second moyen de passage d'air (25) et conduire l'air d'admission filtré à l'humidificateur lorsque l'humidificateur est monté sur le socle (12) et introduire l'air d'admission filtré humidifié au premier moyen de passage d'air (16, 18) lorsque l'humidificateur est monté sur le socle.
